Europäisches Patentamt

European Patent Office

Office européen des brevets

(19)

(11) Publication number: **0 393 868**
**A2**

## (12) EUROPEAN PATENT APPLICATION

(21) Application number: **90303468.4**

(22) Date of filing: **30.03.90**

(51) Int. Cl.5: **G01N 33/543, C12Q 1/00**

(30) Priority: **13.04.89 US 337300**

(43) Date of publication of application:
**24.10.90 Bulletin 90/43**

(84) Designated Contracting States:
**AT BE CH DE DK ES FR GB GR IT LI LU NL SE**

(71) Applicant: **GENERAL BIOMETRICS INC.**
**11199 Sorrento Valley Road**
**San Diego, California 92121(US)**

(72) Inventor: **Kiehl, Bryan L.**
**10797 Brookview Lane**
**San Diego, California 92131(US)**

(74) Representative: **Williams, Trevor John et al**
**J.A. KEMP & CO. 14 South Square Gray's Inn**
**London WC1R 5EU(GB)**

(54) **Immunoassay system.**

(57) The present invention is in an immunoassay system and process for detecting analytes and is illustrated for detecting specific antibody presence in a specimen in a short period of time as in a non-laboratory setting, such as a doctor's office. The system includes a support whereon one or more dots of a specific purified antigen to be tested for are spotted and the area around that spot or spots is preferably blocked. The support is for sequential incubations of approximately four (4) minutes each in reagent vessels containing: a specimen consisting of a diluent and a drop or drops of plasma, serum or whole blood; a high ionic strength wash solution; an enzyme conjugate; a second wash solution containing, depending upon the test procedure, a high or low ionic strength solution; and an enzyme substrate that produces a coloration of the antigen-antibody binding site of an antibody-antigen. The system of the present invention employs a high ionic strength solution as the first wash solution of from 0.25 M to saturation for removing interfering substances, presumably those substances having lower binding affinities, and for increasing sensitivity.

The system reagents are preferably tabletized in the manufacture and are reconstituted just prior to use. Applicant has successfully combined dry and tabletized, as the enzyme substrate reactant, a compound of 5-bromo-4-chloro-3-indolyl phosphate (BCIP) and P-nitro blue tetrazolium chloride (NBT).

Fig. 1

EP 0 393 868 A2

# IMMUNOASSAY SYSTEM

## BACKGROUND OF THE INVENTION

### Field of the Invention

The present invention relates generally to assay systems and processes for detection of analytes and in particular is applicable to immunoassays.

### Prior Art

In the practice of certain immunoassay procedures that provide for antigen-antibody detection it is often the case that substrates present in the serum may cause false interpretations. Particularly, background interference and/or low sensitivity have been found to be a problem where the specimen and/or conjugate steps are to be performed in a short period of time, as say for example, less than fifteen (15) minutes per step. Such has been noticed in earlier United States patent applications that are assigned to the assignee of the present application entitled, "Solid Phase Enzyme Immunoassay Test Apparatus and Process for Detecting Antibodies" filed April 21, 1986, Serial No. 857,643; and "Improved Solid Phase Enzyme Immunoassay Test Apparatus and Process for Detecting Antibodies", filed January 11, 1988, Serial No. 142,013. Each of these antibody detection processes are similar to an enzyme-linked immunosorbent assay (ELISA), and provide reactant incubation steps that are each performed in approximately five (5) minutes.

Both of the above set out patent applications contemplate a distilled or light salt wash of approximately 0.1 M NaCl between incubation steps as do the references cited in the applications and/or as have been cited by the Patent Office in prosecution thereof. Prior to the present invention it was accepted that high ionic strength solutions were typically not used in immunochomical assays as ideal binding affinities for antigen-antibody reactions had, after extensive investigation, been determined to be at isotonic concentrations of approximately 0.15 M. Further, where slightly higher ionic strength solutions are used in an antibody reagent, such as in an immunochemical staining system, such resulted in a diminishment of assay sensitivity. Also, it was observed that use of high salt concentrations for clarification after both the specimen and conjugate steps resulted in unacceptable background interference when using a membrane. It was also observed that high salt wash could be used in an immunofluorescence (IFA) technique without background interference. To the present process, it was state of the art in immunochemical assays to use the same wash reagent after both specimen and conjugate steps and accordingly, a use of two different clarification solutions within the assay system was not formerly investigated.

That high ionic strength solutions have not been employed in the wash steps of earlier immunoassay systems is shown in: a United States patent to Marks, No. 4,774,177, that teaches a tap water rinse between incubations; a United Kingdom patent application, No. 2,099,578A to Gordon that teaches a wash in TBS solution; and in the above-cited United States patent application for a "Solid Phase Enzyme Immunoassay Test Apparatus and Process for Detecting Antibodies", Serial No. 857,643, that utilizes distilled water in the washing steps. Where, as set out in the cited patent application for an "Improved Solid Phase Enzyme Immunoassay Test Apparatus and Process for Detecting Antibodies", Serial No. 142,013, a low ionic solution has been employed as the wash solution, that solution is preferably a 0.1 M or less Nacl solution. Accordingly, it is clear that, prior to the present invention, solutions having high ionic concentrations have not been utilized as wash solutions, and accordingly the present invention has obtained an unexpected result. The present invention has been found to be applicable for practice in the wash step or steps of an enzyme-linked immunoassay (ELISA) as well as a practice of a traditional indirect fluorescent assay (IFA) and anticomplement immunofluorescent assay (ACIF) that detects antibody presence against several antigen, including anti-Epstein-Barr virus viral capsid antigen, Epstein-Barr virus nuclear antigen, IgG anti-CMV and IgM anti-cytomegalovirus. Also, the utilization of a high ionic strength solution for the wash steps of a western blot type assay is believed to be desirable, based on the similarities between a western blot assay and the (ELISA) type assay as set out later herein.

Additional to the utilization of a high ionic concentration for a wash solution, the present invention further includes a utilization of certain reactive materials that are combined in tabletized form and are reconstituted

2

for use as an enzyme substrate reactant. The above cited patent application in an "Improved Solid Phase Enzyme Immunoassay Test Apparatus and Procedure for Detecting Antibodies", Serial No. 142,013, teaches a tabletizing of absorbed materials that are appropriately dried, blended and tabletized according to standard tabletizing procedures including mixing, molding and stamping. The individual tablets to be reconstituted for use with an appropriate diluent. The present system is an improvement thereon and is directed to preparation of an enzyme substrate reactant that will exhibit a long shelf life and can be easily reconstituted.

## SUMMARY OF THE INVENTION

It is a principal object of the present invention in an improved immunoassay system to provide a system for reducing background interference.

Another object of the present invention is to provide increased sensitivity of the improved assay system.

Another object of the present invention is to provide an immunoassay system for quickly detecting antibody presence in specimens.

Another object of the present invention is to provide, for use in an immunoassay system, a high ionic strength solution for washing a test strip after a specimen step, for limiting background interference and/or improving sensitivity of an antigen-antibody reaction.

Still another object of the present invention is to provide, in tabletized or dry packaged form, a stable enzyme substrate reactant for use in an assay procedure.

Still another object of the present invention to provide a high ionic strength solution as a wash solution for reducing background interference for use in practicing an immunoassay system.

The present invention is for use in an immunoassay system and its use is illustrated in an enzyme-linked immunoassay procedure. Such procedure can be practiced to detect the presence of a single or a plurality of different antibodies in a single specimen. The described immunoassay procedure that incorporates the present invention is essentially like an enzyme-linked immunosorbent assay (ELISA) procedure. Such procedure preferably utilizes a rigid support that maintains a membrane whereon an array of purified antigens are dot blotted and dried. The procedure described herein involves reconstituting a tabletized or dry packaged specimen diluent in a vessel wherein a drop or drops of either plasma, serum, or whole blood, with or without a coagulant, are mixed and the membrane maintained on the rigid support is incubated therein for approximately four (4) minutes. In that incubation, specific antibodies as are present in the specimen will bind with like specific antigen dots on the membrane. After this specimen step the stick support is rinsed to remove the diluent and specimen mix, and the membrane is washed. The wash is performed in a vessel containing a high ionic strength solution, of from 0.25 M to saturation, and the membrane is allowed to stand therein for approximately four (4) minutes. This high ionic strength solution provides for removal of interfering substances in and around the specimen to eliminate interference. Following the wash step, the membrane on the stick support is incubated in a vessel containing a conjugate solution preferably consisting of a non-interfering protein, such as an alkaline phosphatase conjugated goat anti-human immunoglobulin. The conjugate solution is preferably reconstituted from a tabletized state but may be packaged in a dry state in a waterproof container, which incubation is for approximately four (4) minutes. The stick support is again washed but preferably in a lesser ionic strength solution or water and is allowed to stand therein for approximately four (4) minutes.

Following the wash between the specimen and conjugate step, the solid support and membrane are incubated for approximately four (4) minutes in a vessel containing an enzyme substrate reactant of the present invention. Which enzyme substrate reactant is suitable for any assay system that employs an alkaline phosphatase. The described procedure is useful also for a western blot system or general chemistry procedure.

Following the enzyme step the stick support with membrane is rinsed, dried and read. In that reading, a bluish violet coloration of an antigen dot indicates the presence of a like antibody binding to that antigen in the specimen. Which reading is facilitated by the high ionic solution washes having eliminated background interferences and/or improved sensitivity, and providing, thereby, an enhanced coloration to each such dot. Preferably, the procedure is conducted at a constant temperature of approximately forty-five (45) degrees.

## BRIEF DESCRIPTION OF THE DRAWINGS

3

These and other objects and features of the present invention in an improved immunoassay system will become more fully apparent from the following description in which the invention is described in detail in conjunction with the accompanying drawings.

Fig. 1 is an exploded perspective view of a stick support consisting of a solid support that includes a membrane mounted thereto, which solid support has spaced holes exposing a section of membrane therethrough with a dot of a purified antigen immobilized thereon and showing a blocking agent applied to the membrane area around that dot;

Fig. 2 is a flow schematic of a practice of an enzyme-linked immunosorbent assay on the stick support of Fig. 1 for determining specific antibody presence; and

Fig. 3 is a view of the third test vessel of Fig. 2, that is shown as receiving, for reconstitution, a tabletized enzyme substrate reactant consisting of (BCIP) and (NBT).

## DETAILED DESCRIPTION

### Procedure

The present invention is for practice with an immunoassay system for detecting antigen and/or antibody presence in a specimen diluent. The procedure discussed herein for illustrating the present invention is similar to a procedure known as an enzyme-linked immunosorbent assay (ELISA) utilized for detecting antibody presence. The present invention is hereinbelow discussed with respect to application of an (ELISA) type assay for specific antibody detection in a human serum specimen and involves binding at least one dot of an antigen on a membrane. The membrane, is preferably nitrocellulose and is mounted to a rigid support that is arranged for sequential incubations. After application of the antigen spot, the strip is placed into a blocking solution consisting of material to render the remaining membrane material non-reactive. The membrane is preferably nitrocellulose and is mounted to a rigid support, this apparatus is subsequently referred to as the assay strip. After application of the antigen spot(s), the assay strip is placed into a blocking solution consisting of a material to render the remaining membrane nonreactive. In this case, the blocking reagent is preferably ten (10) percent powdered, non-fat milk in water. After drying, an assay may be performed with the assay strip. In this case the assay was performed by first adding either serum or heparinized whole blood to a specimen diluent and incubating the assay strip therein, followed by washing the assay strip in a high ionic strength reactant, an incubation of the assay an enzyme conjugated anti-immunoglobulin reactant, followed by washing the assay strip in a low ionic strength wash solution, and finally incubating the assay strip in an enzyme substrate reactant. The assay was performed at forty-five (45) degrees centigrade (C) although some assays may be performed at room temperature. Each incubated with rinses in low ionic strength wash solution therebetween was for four (4) minutes. Use of a high ionic strength solution wash between the specimen and conjugate steps, is to lessen interference, insuring that a color presence on the antigen spot as is present after the enzyme substrate incubation step relates to the amount of bound antibody.

### Wash Step Theory

The basic concept for use of the high ionic strength wash is that substances, in this case substances in the serum, can be dissociated as the ionic strength of a solution is increased. Because of antigen-antibody interaction is a high affinity bonding, within an immunoassay higher ionic strength washes may be used to remove materials which bind with less affinity. These interfering substances may occur in two general forms.

First, a material may be bound to the antibody which is to be measured. If this material is large and complex, subsequent detection by the conjugate may be sterically hindered. It is known that the complement protein, CIQ, a large, complex protein, binds with low affinity to antibody while in serum. A solution with approximately 0.5 M ionic strength will remove most of this protein from the antibody to be detected. By this removal more binding sites for the conjugate are exposed, thus significantly increasing sensitivity. The cause for the increase in sensitivity can only be speculated, but it has been conclusively demonstrated that the high ionic strength solution increases sensitivity. Also, it has been demonstrated that the increase in sensitivity is significantly more than what increase might be expected from the diminishment of background interference as could result from a use of high ionic strength wash alone.

Second, a material may be bound to other than the antibody which is to be measured. This attachment may be made to the nitrocellulose membrane outside the spotted dot region, within the dot region, or may attach to the antigen via a specific or non-specific mechanism. To remove these unwanted substances, a wash with a high ionic strength has been used. After such wash between the specimen and enzyme incubation steps, background interference was found to be lowered, the unwanted background material exhibiting a significantly lower binding affinity than that of the desired antigen-antibody interaction.

In the present case, the cytomegalovirus (CMV) antigen illustrates both effects. It is well known that one or more (CMV) antigens each bind to antibody. Unlike the high affinity bond of the antibody specifically directed against the (CMV), the virus protein propensity for the antibody exhibits less binding affinity. It is therefore possible to remove the non-specific, bound antibody by selective use of a wash solution of high ionic strength. In addition, when serum is removed from an individual, the first component of the complement attached to antibody in the serum. This complement protein is complex and ostensibly exhibits stearic interference. Therefore, in the case of (CMV) when approximately a 0.5 M wash solution is used after the specimen step, it was observed that a significant increase in sensitivity and a decrease in specificity was produced. The degree of observed change cannot be attributed to only one effect and accordingly demonstrates that high ionic strength washes both diminish background interference and increase sensitivity.

Practice

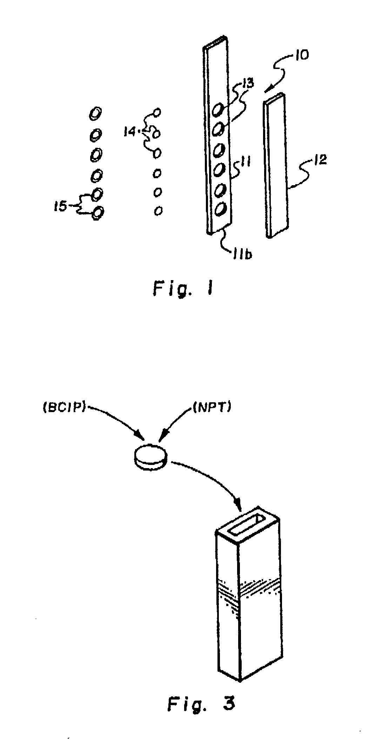

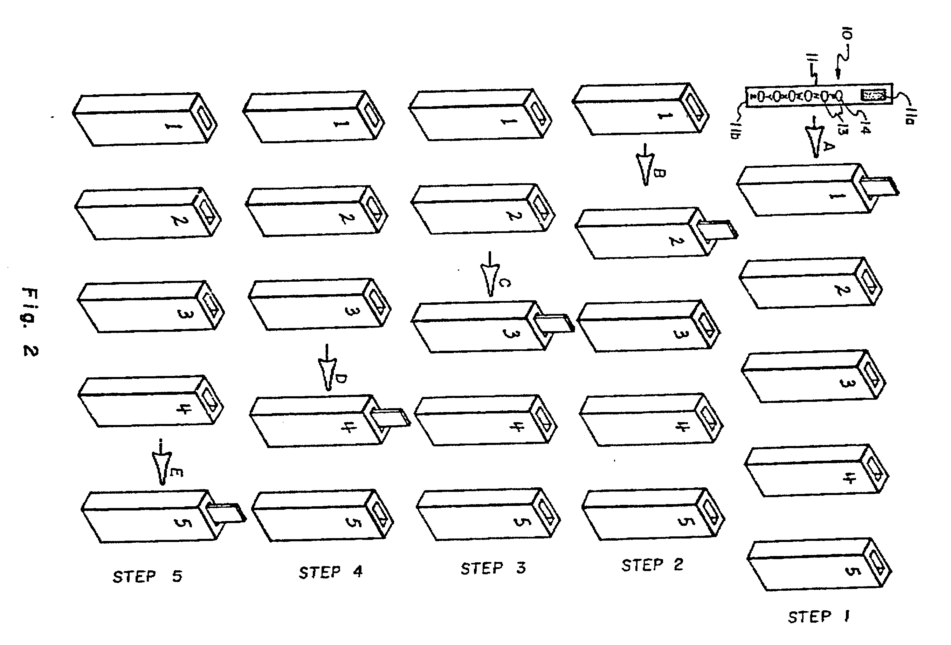

For practicing the preferred (ELISA) like procedure the present invention, as shown in Fig. 1, employs a stick support 10 for sequential incubation in the reactants. The stick support 10 includes a support 11, that can be plastic or like inert material to provide a rigid support for a membrane 12, that is preferably nitrocellulose, and is fixed thereto. Which support 11 and joined membrane 12 are a thin, narrow rectangular section, the support 11 to be held by an operator on one end 11a, for dipping sequentially in a reactant vessels 1, 2, 3, 4, and 5, as shown in Fig. 2. Windows or holes 13 are formed at spaced intervals along support 11, to display the membrane 12 therethrough. Small amounts of approximately one (1) microliter each of antigen samples 14 are spotted (immunodotted) on the membrane 12, so as to be approximately centered within each window or hole 13. Preferably, the surface of the membrane around each antigen dot 14 is blocked, illustrated as center holed disks 15 in Fig. 1. Membrane blocking is employed to cover the nitrocellulose surface around the antigen dot with a non-interfering protein, or other like compound. This blocking is to limit or prevent a non-specific binding of immunochemical compounds thereto as the antigen dots 14 are exposed to in sequential reagent incubations. The membrane blocking is such as to minimally effect the antigen dots 14, and is practiced to limit the high affinity that most membranes, such as nitrocellulose, exhibit to non-specific absorption of a wide range of materials. In the present invention, to provide this membrane blocking, the stick support 10 below end 11a is preferably immersed in a solution containing ten (10) percent non-fat dry milk in water for approximately forty-five (45) minutes at ambient temperature. Other membrane blocking solutions, however, may be so employed to include, solutions of albumin, caesin, gelatin, detergents, amino rich compounds, and the like. Also, the time and temperature that the membrane is exposed to the blocking agent may be varied depending on the membrane employed and the desired level of acceptable sensitivity or interference. Further, membrane blocking may be omitted entirely for some applications where a lower level of sensitivity is acceptable. Also, a utilization of the preferred high ionic strength wash solution provides for limiting background interference for many immunoassay procedures including an (ELISA) typen procedure, and accordingly, for some such procedures, blocking may not be necessary.

Following membrane blocking, the stick support 10 is briefly rinsed, preferably in distilled water, and is allowed to air dry at ambient temperature. Other rinse solutions may also be employed such as buffered or detergent containing solutions where greater levels of sensitivity for a particular test configuration are desired and other than ambient temperatures may be used to either accelerate or slow the process. Blocked stick supports 10 can then be packaged and stored until used. They may be packaged in any suitable container or not packaged at all depending on their anticipated use and may be stored either at below freezing, refrigerated or ambient temperature.

Preferably, as set out above, a number of windows or holes 13 are formed in support 11, exposing sections of the membrane 12 therein. The exposed membrane is for receiving different antigens that are purified and spotted therein, which windows or holes are identified as W, X, Y, and Z in Fig. 2. Also, windows or holes 13 identified with letters P and N, can receive controls dotted thereon.

A second window from the support top 11a is shown labeled as N. This window or hole preferably contains a negative control spotted therein. Such control preferably consists of a buffer solution as is used for the solubilization/dilution of all the other samples that are spotted in the lettered windows, or can be a negative control antigen such as a cellular extract of uninfected cells as used for antigen production. Finally a top window or hole identified as P, can contain a positive control.

Such positive control is to verify that all reagents are functioning properly and that the test procedures have been correctly executed. The control will also verify that the five (5) reaction vessels, shown in Fig. 2, are sufficiently reconstituted to a required volume to successfully perform the test procedure. In practice, this window has been spotted with a solution of purified human immunoglobulin G (IgG) as the positive control. It should be understood that solutions other than those described may be used as the positive and negative controls or these controls may be omitted entirely and the windows or holes 13 designated as P and N could be dispensed with or used for other purposes within the scope of this disclosure. The windows or holes 13, identified as W, X, Y, and Z, in practice, are preferably spotted with various purified antigens that are each for a certain antibody to be tested for. The total number of antibodies to be tested for, of course, determines the number of windows or holes 13 as are needed. In practice, as set out in the examples below, a stick support 10 that incorporates six windows, two for the positive and negative controls with the other four (4) windows spotted with various purified antigens, has been used successfully.

Once the membrane 12 surface that shows through windows or holes 13 has been spotted with the appropriate solutions these spots 16 are allowed to air dry at ambient temperature. Such drying usually requires several minutes though longer drying times may be employed without noticeable effect. Also, temperatures other than ambient may be employed without noticeable effect.

Fig. 2 illustrates a preferred practice of an enzyme-linked immunoassay procedure utilizing the improvements of the present invention. The practice includes successive incubations of stick support 10 of Fig. 1 in containers or vessels 1, 2, 3, 4 and 5 with rinses between incubations. To detect a specific antibody from a clinical sample the stick support 10, prepared as set out above, is successively incubated at forty-five (45) degrees centigrade (C) for approximately four (4) minutes in each of five (5) separate immunochemical solutions that are contained, respectively, in vessels 1, 2, 3, 4 and 5. While the steps shown in Fig. 2 are preferably performed at the stated forty-five (45) degrees centigrade (C), though other incubation temperatures, including room temperature, and times may also be employed to effect a speeding up or slowing down of an incubation, within the scope of this disclosure. Further, it has been found in practice that for some assays, for example, assays for Rubella and Toxoplasma, that a second wash, illustrated as vessel 4, may be eliminated within the scope of this disclosure.

The process of the present invention is preferably performed in small plastic containers or vessels, each containing a reagent. Each container or vessel is to receive the membrane, at the end 11b of the stick support 10, fitted therein. Fig. 3 illustrates the mixing of a tablet of a preferred enzyme substrate reactant of the present invention in vessel 5. Preferably, each reactant tablet for vessels 1, 2, 3 and 5 is a 100 mg table, that is added to the empty dry container and, is reconstituted with approximately 2 ml water. Vessel 4 is preferably filled with 10-50 ml of water. It should, however, be understood that, within the scope of this disclosure, the reagent components can be mixed dry and left in a powder form and be reconstituted as described. By fitting of the stick support membrane end 11b into and moving it appropriately, the contents of each vessel are sufficiently mixed to re-suspend the reagent.

For the present process the reagent and wash tablets are preferably prepared from the materials described below. In that preparation, appropriate materials are blended, which blends are then preferably tabletized according to standard tabletizing procedures common to the tabletizing industry to include mixing and compression molding or stamping. The tablets are preferably prepared from bulk quantities of the chemical reagents as listed below, the recipes to produce approximately one (1) one hundred (100) mg tablet of each reagent. Of course, in practice, the tablets are preferably compounded in large batches and the weights of materials are accordingly increased proportionally for the number of tablets to be produced. Additionally, a variety of modifications of the reagent recipes are possible within the scope of this disclosure. Such modifications may be employed to enhance tablet disintegration time, stability, cost, and activity of the critical immunochemical reagents. Because of their shelf life, packaging and handling ease and low production cost tabletized or dry powder reagents are more desirable than lyophilized or liquid reagents.

Prior to the present invention it was believed that preferred enzyme substrates, 5-bromo-4-chloro-3-indolyl phosphate (BCIP) and P-nitro blue tetrazolium chloride (NBT), were unstable in combination and further that (BCIP) was insufficiently soluble in water for optimum performance and accordingly would not have an adequate shelf life when tabletized. Where conventional formulations obtained solubilization of (BCIP) and/or (NBT) when dissolved in an organic solvent and water, it was found that this mixture, when dried to a powder form and mixed, would continue to be reactive in its dry format. This activity unacceptably reduced the stability of the reactant. From observation it was determined by the present inventor that although (BCIP) and/or (NBT) is much less soluble in water than in conventional organic solvents (such as N, N, dimethyl formanide), and water, it is nevertheless sufficiently soluble for optimum reactivity within an assay. In addition, it was observed that up to tenfold excess (BCIP) or (NBT) did not interfere with the assay. Accordingly, it was determined that when the two substrates (BCIP) and (NBT) are mixed in their dry chemical state, without first being dissolved and then dried, they can be combined into a stable powder combination that is suitable for dry packaging or formation into tablets. This dry packaged or tabletized state has been found to be stable when prepared and packaged, as set out above, and the compound will remain stable during the shelf life required of the other reactants. Which shelf life has been determined to be in excess of eighteen (18) months for an assay system that will be commercially available.

Where the above enzyme substrate reactant has been successfully dry packaged and tabletized for reconstitution as an enzyme developer for the described (ELISA) like immunoassay process set out herein, it should be understood that this formulation is not limited to the described immunoassay process only. This formation can be universally used as an enzyme substrate reactant in any assay system, such as general chemistry assays for alkaline phosphatase and western blot assays employing alkaline phosphatase enzyme, within the scope of this disclosure.

The preferred constituents of the reagent tablets for practicing described (ELISA) like immunoassay procedure for reconstitution in each reactant container of Fig. 2 are as follows:

| Tabletized Sample Diluent | |
|---|---|
| Vessel #1 | |
| Sodium chloride | 17.000 mg |
| Sodium phosphate, monobasic | 0.690 mg |
| Sodium phosphate, dibasic | 4.170 mg |
| Bovine serum albumen | 10.000 mg |
| Magnesium stearate | 0.940 mg |
| Microcrystalline cellulose | 66.200 mg |

| Tabletized High Ionic Strength Wash | |
|---|---|
| Vessel #2 | |
| Magnesium stearate | 0.99 mg |
| Color, FD&C #3 | 0.01 mg |
| Microcrystalline cellulose | 40.50 mg |
| Sodium chloride | 58.50 mg |

| Tabletized Conjugate | |
|---|---|
| Vessel #3 | |
| Sodium chloride | 17.000 mg |
| Sodium phosphate, monobasic | 0.690 mg |
| Sodium phosphate, dibasic | 4.170 mg |
| Magnesium stearate | 0.951 mg |
| Magnesium sulfate | 0.241 mg |
| Color, FD&C yellow #6 | 0.050 mg |

Wash

Vessel #4

Water with or without preservatives or sterilized.

| Tabletized Developer | |
| --- | --- |
| Vessel #5 | |
| Sodium chloride | 11.680 mg |
| Magnesium sulfate | 12.040 mg |
| Color, FD&C blue #1 | 0.010 mg |
| Microcrystalline cellulose | 46.820 mg |
| 5-bromo-4-chloro-3-indolyl phosphate (BCIP) | 1.330 mg |
| p-nitro blue tetrazolium chloride (NBT) | 2.640 mg |
| Trizma HCL | 1.680 mg |
| Trizma base | 22.800 mg |
| Magnesium stearate | 1.000 mg |

In practicing the described immunoassay process, the first of the five (5) containers or vessels shown in Fig. 2, numbered 1, is to contain the sample diluent that is reconstituted from a tablet form, preferably using a 2 ml solution of water. The diluent is mixed by quick up and down motions of the stick support, so as to thoroughly mix a small quantity of a clinical sample of as little as one microliter, of plasma, serum or whole blood. Alternatively, an anti-coagulant may be included as part of the reagent or may be added to the reconstituted diluent. The preferred reconstituted diluent for vessel 1 is as set out above. Additionally, within the scope of this disclosure, other solutions may also be employed as the diluent such as, for example, Tris-saline, detergent containing solutions, and the like, provided that such other solution minimizes non-specific interactions of the immunochemical reagents.

In the specimen step, illustrated as arrow A in Fig. 2, the stick support 10 is allowed to incubate for approximately four (4) minutes in the diluent and clinical sample and is then swished back and forth through a rinse solution for a few seconds.

After the specimen step where the stick support 10 is incubated in the presence of the clinical smaple and sample diluent and the rinse, the stick support is positioned in a vessel 2, as illustrated in step 2 of Fig. 2. Vessel 2 contains a high ionic strength solution. In practice, a preferred tablet or dry powder containing sodium chloride and inert materials is reconstituted with water to provide approximately a 0.5 M solution. Illustrated as arrow B, the wash vessel receives the stick support 10 that is moved up and down therein and is then allowed to stand in the solution for approximately four (4) minutes. The effect of a utilization of a high ionic strength wash solution of 0.25 M to saturation and theory is set out hereinabove under "Wash Step Theory".

Although an ionic strength of around 0.25 M has heretofore been used in an immunochemical staining system, known as immunoperoxidase histological staining, use of a high ionic strength solution for an assay involving animal protein like that described was heretofore believed to be impractical as it was observed that as the ionic strength increased during the specimen and/or conjugate step, the assay sensitivity diminished. Also, it has been observed that a use of high salt concentrations for clarification after both the specimen step and conjugate in the (ELISA) steps caused unacceptable background interference. Heretofore where the same wash solution has uniformly been employed for wash steps between specimen and conjugate and conjugate and substrate steps, it was not thought to employ a high ionic strength wash solution between the specimen and conjugate steps with a water wash between the conjugate and substrate steps as practiced by the present invention for such (ELISA) type procedure.

Accordingly, as set out above, for the described immunoassay where nitrocellulose is used as the membrane, it has been found that a high ionic strength solution may only be used to wash the sample after it has been reacted, and that low ionic strength solutions, below 0.25 M, preferably water alone, should be used after the conjugate reaction step to avoid a loss of antigen-antibody binding affinity. It is believed that this will also be the case in a practice of a western blot type assay that is similar to the process described herein.

For other assays, however, such as direct and indirect immunofluorescent assays, high ionic strength solution washes are appropriate after both the sample and conjugate steps. This has been found in practice to be true for both an indirect fluorescent assay (IFA) measuring IgG and IgM antibody presence against cytomegalovirus and Epstein-Bar virus; and an anticomplement immunofluorescent assay (ACIF) detecting antibody presence against Epstein-Barr virus nuclear antigen. In both procedures, background fluorescence was significantly reduced, and the aesthetic improvement was substantial. In practice, in several of the (IFA) procedures, specimens which were difficult to interpret due to their low level fluorescent response were clearly interpreted as negative samples. These samples were confirmed to be true negative samples by use of alternative assay method.

It is believed that a use of a high ionic strength solution after the specimen step and prior to the conjugate step in all immunochemical assays will yield a significant improvement in the sensitivity and/or specificity of the overall reaction. It is also to be anticipated that other chemicals such as other salts, for example, potassium chloride, calcium chloride, lithiumchloride, sodium, sulphate, sodium phosphate or chaotropic agents, urea, thiocyanate will provide similar improvements to assay performance.

High ionic strength clarification of the specimen may be adapted, but is not exclusive, to both covalent and non-covalent bound analytes to a solid support system. These solid support systems include, but are not restricted to the following: (1) nitrocellulose, (2) polystyrene, (3) nylon, and (4) paper. An anticipated common use for this process is a western blot analysis of analytes using nitrocellulose and enzyme immunosorbent assays (ELISA) with either polystyrene microliter strips, plates or beads.

Following the first wash in vessel 2, the stick support 10 is incubated for approximately four (4) minutes in container 3, illustrated as arrow C. This container preferably contains a reconstituted conjugate that is produced by mixing approximately 2 ml of water with the tabletized or dry chemicals conjugate, as set out above. It should, however, be understood that, conjugates of other specificities and different enzyme (e.g. peroxidase, glucose oxidase, etc.) are also within the scope of this disclosure. During this incubation the enzyme labeled conjugate will bind to clinical sample antibodies as are present in the specimen diluent and have bound to specific antigen dots or spots. Additionally, the conjugate will also bind to the positive control spot, if utilized, during this incubation.

The incubation of the stick support 10 in vessel 3 is followed by another rinse, that is illustrated as arrow D. This rinse for an enzyme-linked immunoassay like the described procedure is not conducted in the high ionic strength solution contained in vessel 4, but is preferably conducted in a solution having a low ionic concentration, in this case is preferably water. However, for a direct or indirect immunofluorescence assay, the described high ionic strength solution may also be utilized for this wash step. The strip support to stand in vessel 4 for approximately four (4) minutes, which vessel preferably contains from 10 to 50 ml of water.

Following the second wash, the stick support 10 is incubated in the fifth vessel, illustrated as arrow E, that contains an enzyme substrate reactant that is to react with the conjugate in such a way as to visually reveal the presence of said conjugate bound to the coupled antigen-antibody. The preferred enzyme substrate is that discussed above that has been reconstituted by mixing the dry tabletized or powdered (BCIP) and (NBT) in water. During this incubation the preferred alkaline phosphatase will interact with the NBT/BCIP substrates. The product of this enzymatic interaction, is the production and deposit of insoluble colored precipitates on the nitrocellulose membrane 11 at the site of the bound enzyme conjugate dot 14. This deposit and its tight binding to the membrane produces, on the dot 14, a blue-violet color against a white background of the nitrocellulose membrane 12. This incubation may be followed with a brief rinse in water solution or distilled water.

Colored spots that have appeared within one or more of the windows or holes 13, after the fifth incubation, indicate the presence of a specific antibody for that specific antigen in the clinical specimen. The absence of such colored spot indicates that such specific antibody is not present. Further, as a verification of the validity of the test results, examination of the positive and negative control windows P and N should show a presence of a colored spot in the positive control window with no detectable spot in the negative control window. This indicates that all test reagents worked properly at the time of testing and that the proper procedures for executing the test were followed. Results other than these for the two controls invalidates the test.

In practice, the effects of a utilization of a high ionic strength solution as the wash reagent between specimen and enzyme conjugate steps in testing for cytomegalovirus (CMV) demonstrated that, in addition to the increase in sensitivity and specificity, the incorporation of a 1.0 molar sodium chloride wash solution into an immunofluorescence assay was yielded results which differed from those observed using two other

9

widely used methods. Two hundred fifty-five (255) human serum samples were tested for the presence of human antibody directed toward (CMV) using a latex assay, a standard immunofluorescence assay (IFA), and the immunofluorescence assay using 1.0 M sodium chloride. As shown in Table 1, twenty six and four tenths (26.4) percent more positives were observed in the (IFA) incorporating salt when compared to the latex assay results. Furthermore, twenty-one and seven tenths (21.7) percent less positives were observed int he (IFA) incorporating salt than seen with a standard (IFA) using otherwise identical reagents.

Table I

|  | LATEX | HIGH SALT IFA | STANDARD IFA |
|---|---|---|---|
| Positives | 140 | 177 | 226 |
| Negatives | 115 | 78 | 29 |

While the workings of the present invention has been described with respect to a practice of an enzyme-linked immunosorbent assay (ELISA) like procedure for detecting antibodies in a clinical specimen, it should be apparent that this disclosure also relates to immunofluorescence assays and like assays, where it is appropriate to reduce background interference by the utilization of a high ionic strength solutions as the wash solutions, including blood analysis generally, and accordingly, the present disclosure is made by way of example only and that variations are possible within the scope thereof without departing from the subject matter coming within the scope of the following claims, which claims I regard as my invention.

## Claims

1. An immunoassay system for detecting from an animal serum protein an antigen-antibody reactant comprising, a support means for maintaining a purified antigen or antibody sample thereon; a first vessel means for containing a clinical diluent for receiving an animal protein specimen wherein said support means is incubated for a sufficient period of time for a specific antibody or antigen from the specimen to bind to the sample antigen or antibody; a second vessel means for containing a wash solution of a high ionic strength of 0.25 M to saturation for receiving, following said specimen incubation, said support means that is washed and left to stand therein a sufficient time period for that wash solution to selectively prevent non-specific interaction at the antigen-antibody reaction site and to remove interfering substance at the reaction site, thereby increasing assay sensitivity; a third vessel means containing an enzyme conjugate for receiving, following said wash, said support means incubated therein for a sufficient period of time for said enzyme conjugate to bind to the specimen antigen-antibody binding site; and a final vessel means for containing an enzyme substrate for receiving said support means incubated therein for a sufficient period of time to where the enzyme substrate reacts to the enzyme conjugate and provides a colored precipitate on said support means at the site whereat the enzyme conjugate is bound to the specimen antigen-antibody.

2. An immunoassay system as recited in Claim 1, further including a fourth vessel means for containing a wash solution that receives said support means for washing and standing therein between the incubations in the third and final vessel means.

3. An immunoassay system as recited in Claim 2, wherein the period of time for each incubation and washing is four (4) minutes.

4. An immunoassay system as recited in Claim 1, wherein the reagents contained in each of the vessel means are each reconstituted from a tabletized form.

5. An immunoassay system as recited in Claim 4, wherein the reagents are separately reconstituted with an aqueous solution.

6. An immunoassay system as recited in Claim 4, wherein the enzyme substrate consists of a combination of 5-bromo-4-chloro-3-indolyl phosphate (BCIP) and P-nitro blue tetrazolium chloride (NBT) that are combined dry into a tablet form and are reconstituted in an aqueous solution.

7. An immunoassay system as recited in Claim 6, wherein an excess of (BCIP) and (NBT) are combined to provide, when reconstituted with an aqueous solution, a sufficient reactive product to serve as the enzyme substrate.

8. An immunoassay system as recited in Claim 1, wherein the vessels contents are maintained at a constant temperature.

9. An immunoassay system as recited in Claim 8, wherein the vessels contents are maintained at approximately forty-five (45) degrees centigrade (C).

10. An immunoassay system as recited in Claim 8, wherein the vessels contents are maintained at room temperature.

11. An immunoassay procedure utilizing a support that maintains a sample thereon of a specific purified antigen or antibody to be tested for consisting of, incubating the support and sample in a first vessel containing a reagent of a clinical diluent and animal protein specimen; washing the support and sample in a second vessel containing a reagent of a high ionic strength wash solution of between 0.25 M and saturation; incubating the support and sample in a third vessel containing a reagent of an enzyme conjugate; and incubating the support and sample in a final vessel containing a reagent of an enzyme substrate that reacts with the enzyme conjugate to produce a visible colored precipitate at the site of the sample on the support.

12. An immunoassay procedure as recited in Claim 11, further including washing the support and sample between the enzyme conjugate and enzyme substrate incubations in a reagent contained in a fourth vessel.

13. An immunoassay procedure as recited in Claim 12, wherein the support and sample are incubated and washed, respectively, in each vessel for approximately four (4) minutes.

14. An immunoassay procedure as recited in Claim 11, wherein the high ionic strength wash solution in the second vessel is approximately 0.5 M.

15. An immunoassay procedure as recited in Claim 12, wherein the same high ionic strength wash solution is utilized for the wash steps set out as conducted in the second and fourth vessels.

16. An immunoassay procedure as recited in Claim 11, wherein the reagents contained in all the vessels are reconstituted with an aqueous solution from tablets.

17. An immunoassay procedure as recited in Claim 16, wherein the tabletized enzyme substrate is a compound of 5-bromo-4-chloro-3-indolyl phosphate (BCIP) and P-nitro tetrazolium chloride (NBT) that are combined dry for reconstitution with an aqueous solution.

18. An immunoassay procedure as recited in Claim 17, wherein recognizing that (BCIP) and (NBT) are not fully soluble in an aqueous solution without an organic solubilizer, the amount of each of the constituents (BCIP) and (NBT) are selected and combined so as to provide sufficient reactive product.

19. An immunoassay procedure as recited in Claim 12, wherein the vessel contents are maintained at forty-five (45) degrees centigrade (C).

20. An immunoassay procedure as recited in Claim 12, wherein the vessel contents are maintained at room temperature.

21. In an assay that involves producing a chemical reaction on a material that includes an animal protein; a wash solution reagent for selectively removing interfering substances comprising, a 0.25 M to saturation ionic strength or chaotropic solution wherein the material is washed between specimen and conjugate incubations.

22. A wash solution reagent as recited in Claim 21, wherein a 0.5 M ionic strength solution is utilized as the wash solution.

23. A wash solution reagent as recited in Claim 21, wherein the wash solution is for use between both the specimen and conjugate incubations and the conjugate and enzyme substrate incubations.

24. An enzyme substrate organic chemical compound comprising, a compound of 5-bromo-4-chloro-3-indolyl phosphate (BCIP) and P-nitro blue tetrazolium chloride (NBT) that are combined dry for reconstitution utilizing an aqueous solution.

25. An enzyme substrate as recited in Claim 24, wherein the chemicals (BCIP) and (NBT) are combined dry into a tabletized form.

26. An enzyme substrate as recited in Claim 24, wherein the amount of each of the chemicals (BCIP) and (NBT) of the compound are selected to provide, when reconstituted in an aqueous solution, sufficient reactive product to react with an appropriate enzyme conjugate at a site of an antigen antibody reaction so as to produce a color precipitate at that site.

Fig. 1

Fig. 3

Fig. 2